(19) Europäisches Patentamt
European Patent Office
Office européen des brevets

(11) Publication number: 0 126 287 B1

(12) # EUROPEAN PATENT SPECIFICATION

(45) Date of publication of patent specification: **16.06.87**

(51) Int. Cl.$^4$: **C 07 C 59/56,** C 07 C 59/64, C 07 C 57/38, A 61 K 31/19

(21) Application number: **84104201.3**

(22) Date of filing: **13.04.84**

(54) **Fluorinated compounds having analgesic and antiinflammatory activities.**

(30) Priority: **21.04.83 IT 2070583**

(43) Date of publication of application:
**28.11.84 Bulletin 84/48**

(45) Publication of the grant of the patent:
**16.06.87 Bulletin 87/25**

(84) Designated Contracting States:
**AT BE CH DE FR GB IT LI LU NL SE**

(56) References cited:
**DE-A-1 518 528**
**DE-A-2 528 958**
**FR-A-2 295 738**

**The file contains technical information submitted after the application was filed and not included in this specification**

(73) Proprietor: **MEDEA RESEARCH S.r.l.**
**Via Cappuccini, 20**
**I-20100 Milano (IT)**

(72) Inventor: **Bruno, Graziella**
**Via Cappuccini 20**
**I-20100 Milano (IT)**

(74) Representative: **Bianchetti, Giuseppe et al**
**Studio Consulenza Brevettuale Via Rossini, 8**
**I-20122 Milan (IT)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention refers to new fluorinated derivatives of acetic acid having formula I

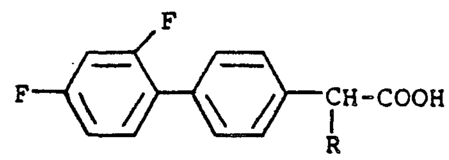

I

wherein R may be an hydroxy, methoxy, ethoxy group or a chlorine atom and pharmaceutically acceptable salts of said derivatives, particularly salts of alkali or alkali-earth metals or salts with basic aminoacids such as lysine or arginine.

The compounds of the invention are endowed with interesting and useful pharmacological properties, particularly analgesic and antiinflammatory activities which make them useful in therapy. Another aspect of the present invention is therefore constituted by pharmaceutical compositions containing as the active ingredient one or more compounds of formula I or of their physiologically acceptable salts, optionally in combination with suitable excipients commonly used in pharmaceutical technique.

The compounds of the invention proved to be less toxic and remarkably more active in comparison with other known antiinflammatory and analgesic agents, such as Naproxen and Diflunisal.

The latter, recently and widely used in clinical medicine and described in U.S. Patent n.3.674.870, shares with the compounds of the invention the 2,4-difluoro-biphenyl group.

Diflunisal, on the other hand, is a salicylic acid derivative and is therefore endowed with structural, chemicho-physical characteristics and, presumably, a mechanism of action, completely different from the ones of the compounds of the present invention, whose activity should be therefore considered completely unexpected and surprising.

FR—A—2 295 738 describes derivatives of biphenylylacetic acids variously substituted by halogen atoms, having analgesic, antiinflammatory and antipyretic activity which, however, appears to be lower than the one of the compounds of the invention as per re according to the classic pharmacological tests on the experiment animals.

The compounds of formula I can be conveniently prepared by reaction of 2,4-difluoro-biphenyl with derivatives of oxalic or glyoxylic acids.

Particularly, the monoethylester of glyoxylic acid chloride can be reacted with 2,4-difluorodiphenyl in the presence of Lewis acids: after reduction of the dicarbonyl compound obtained and hydrolysis of the ester group, the 2-hydroxy-2-[p-(2'', 4''-difluorophenyl)phenyl]acetic acid Ia is obtained, from which it is possible to obtain the other compounds object of the invention by displacement of the hydroxy group with known methods, according to the following scheme:

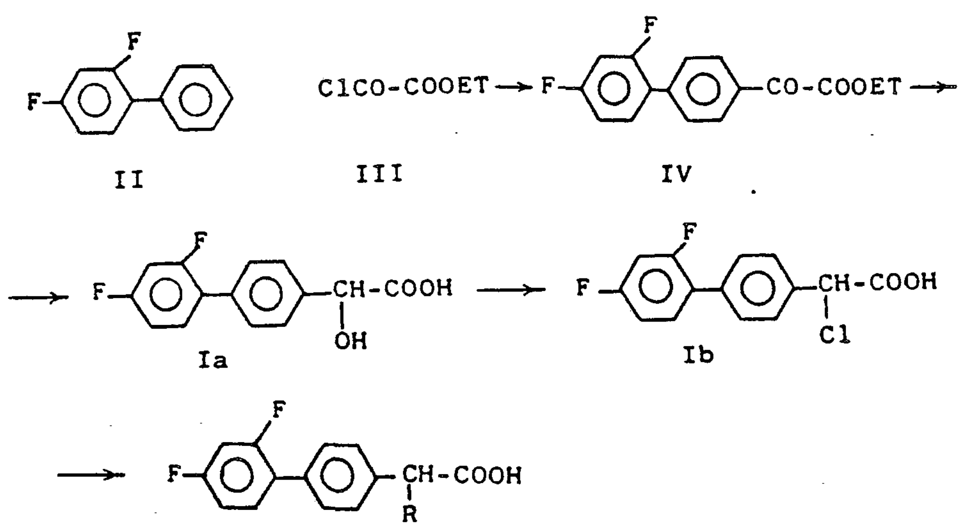


II III IV

Ia Ib

Ic (R = $OCH_3$) Id (R = $OC_2H_5$)

The above scheme should not be considered in a limitative way because it is plain that numerous changes can be carried out by experts in the art: for instance, compounds Ic and Id can be prepared by direct etherification of the ester of the acid Ia, different solvents, reagents and catalysts can be used to carry

out some of the reported transformations, optionally also using phase transfer techniques. The pharmaceutically acceptable salts of compounds of formula I can be obtained by using conventional methods such as precipitation from solvents, crystallizations or lyophilization of aqueous solutions.

The invention comprises the single enantiomers of compounds of formula I which can be obtained either by conventional optical resolution of the racemic mixture or by asymmetric synthesis.

The following non limiting examples further illustrate the invention without limiting in any way the scope thereof.

Example 1

2-Hydroxy-2-[p-(2″,4″-difluorophenyl)phenyl]acetic acid Ia (MR 864)

a) 32 Grams of $AlCl_3$ were suspended in 750 ml of 1,1,2,2-tetrachloroethane and 33 g of ethyloxalylchloride were added thereto, under strong stirring, keeping the temperature between 16 and 22°C.

A clear solution was obtained.

A solution of 45.4 g of 2,4-difluorodiphenyl in carbontetrachloride was dropped in 5—6 hours, always keeping said temperature.

The mixture was stirred for two additional hours and left overnight at room temperature.

The reaction was followed by TLC (dichloromethane as eluent).

The mixture was then cooled to 0°C and slowly poured in water to destroy the red aluminium complex, without exceeding the temperature of 20°C, with stirring.

The phases were separated, washed with water to neutrality and dried on anhydrous $Na_2SO_4$. The solvent was evaporated and the residue distilled at 200°C. 42 Grams of yellow solid still slightly impure were obtained (64.4% yield).

b) The product of the above step a) was dissolved in 500 ml of 95° alcohol, $KBH_4$ was added in portions and the mixture was stirred for 4 hours. 10 ml of acetic acid were added and the mixture was washed, dried and evaporated.

A precipitate, which was discarded because consisting of the corresponding diole deriving from the reduction of the ester group, was obtained with acetonitrile. The filtrate was evaporated and 38 g of a yellow solid were obtained. Yield 89.8%.

c) A yellow solid, soluble in water, cyclohexane and petroleum ether; slightly soluble in toluene, chloroform, carbon tetrachloride, was obtained by saponification with 10% KOH, acidification, filtration, drying, re-crystallization from toluene.

M.p. = 173—174°C.

TLC-eluents: chloroform, cyclohexane, acetic acid 5-4-1.

Example 2

2-Chloro-2-[p-(2″,4″-difluorophenyl)phenyl]acetic acid Ib (MR 881)

10 Grams of 2-hydroxy-2-[p-(2″,4″-difluorophenyl)phenyl]acetic acid were suspended in 60 ml of anhydrous toluene. 7 ml of $SOCl_2$ were added to the suspension under magnetic stirring.

The mixture was left to react at room temperature for two hours and then refluxed for one hour. At the end, the mixture was washed with water and extracted.

The organic phase was dried on anhydrous sodium sulphate, filtered and dried at reduced pressure. The product crystallized from toluene, as white crystalline solid melting at 107—108°C. The product was insoluble in water, soluble in the usual organic solvents, soluble in bicarbonate solutions.

*Elemental analysis* for $C_{14}H_9ClF_2O_2$ (M.W. 282,71)
Calc. % C = 59.47; H = 3.2
Found % C = 59.42; H = 3.16

Example 3

2-Methoxy-2-[p-(2″,4″-difluorophenyl)phenyl]acetic acid Ic (MR 883)

7 Grams of ethyl-2-chloro-2-[p-(2″,4″-difluorophenyl)phenyl]acetate were dissolved in 30 ml of methanol; 1.34 g of sodium methoxyde dissolved in 20 ml of methanol were added to the stirred solution.

The reaction mixture was refluxed for two hours, checked by TLC, dichloromethane as eluent. At the end, the solvent was evaporated at reduced pressure, the crude residue was treated with 100 ml of glacial acetic acid and 50 ml of 37% HCl.

The so obtained mixture was refluxed to complete hydrolysis; the solvent was evaporated, the residue treated with boiling toluene and the insoluble residue was filtered. The toluene solution was dried on anhydrous sodium sulphate, filtered and evaporated at reduced pressure; the crude residue was crystallized twice from cyclohexane.

3.6 grams of white crystalline product melting at 119—121°C were obtained.

The product was soluble in the usual organic solvents.

*Elemental analysis* for $C_{15}H_{12}F_2O_3$ (M.W. 278.26)
Calc. % C = 64.74; H = 4.34
Found % C = 64.47; H = 4.3

The structure of compound MR 883 was confirmed by spectroscopical data.
I.R. Spectrum (recorded in nujol mull, the absorption bands values are expressed in $cm^{-1}$):

| | | | |
|---|---|---|---|
| stretch | O—H | broad band | 3600—3250 |
| stretch | C=O | | 1745 |

*$H^1$ N.M.R. Spectrum* (recorded in $CDCl_3$, internal reference TMS, the chemical shifts values are expressed in δ):

| | |
|---|---|
| 3.4 | (s, 3H, $OCH_3$); |
| 4.8 | (s, 1H, $\underline{CH}$—$OCH_3$); |
| 6.7—7.5 | (m, 7H aromatics); |
| 9.8 | (s, 1H, OH mobile). |

Example 4

2-Ethoxy-2-[p-(2″,4″-difluorophenyl)phenyl]acetic acid Id (MR 936)

The same method for the preparation of the compound MR 883 was used, by using 7 g of ethyl 2-chloro-2-[p-(2″,4″-difluorophenyl)phenyl]acetate in 30 ml of ethanol and adding to the stirred solution 1.7 g of sodium ethylate, dissolved in 20 ml of ethanol.

After hydrolyzing with 37% HCl as already described and after several washings with toluene, the crude product was crystallized from hot cyclohexane; a white crystalline solid was therefore obtained, m.p. = 92°C.

The product was soluble in bicarbonate solution and in the usual organic solvents.

*Elemental analysis* for $C_{16}H_{14}F_2O_3$ (M.W. = 292.272)
Calc. % C = 65.74; H = 4.82
Found % C = 65.68; H = 4.78

The structure of the compound MR 936 is confirmed by spectroscopical data.
*I.R. Spectrum* (recorded in nujol mull, the absorption bands values are expressed in $cm^{-1}$):

| | | | |
|---|---|---|---|
| stretch | O—H | broad band | 3600—3250 |
| stretch | C=O | | 1740 |

*$H^1$ N.M.R. Spectrum* (recorded in $CDCl_3$, internal reference TMS, the chemical shifts values are expressed in δ):

| | |
|---|---|
| 1.25 | (t, 3H, $CH_2$—$\underline{CH_3}$); |
| 3.55 | (q, 2H, $\underline{CH_2}$—$CH_3$); |
| 4.9 | (s, 1H, $\underline{CH}$—$OC_2H_5$); |
| 6.65—7.6 | (m, 7H aromatics); |
| 9.7 | (s, 1H, OH mobile). |

The pharmacological properties of the compounds of the invention, named with the respective abbreviations already reported in the examples, are hereinafter described.

Acute toxicology

A study of the indicative $LD_{50}$ in the rat and the Irwin's test has been carried out on the four compounds. The indicative $LD_{50}$ values are reported in the following table:

| MR 864 | MR 881 | MR 883 | MR 936 |
|---|---|---|---|
| 850 mg/kg i.p. | 850 mg/kg i.p | 2200 mg/kg i.p | 1800 mg/kg i.p. |

It is evident how all the four compounds, and above all MR 883, are less toxic in the rat than Diflunisal which has a $LD_{50}$ p.o. of about 750 mg/kg.

PHARMACOLOGICAL ACTIVITY

Antiinflammatory activity

The antiinflammatory activity of the compounds of the invention was determined according to the carrageenin oedema test in rats, in comparison with equiponderal dosages of Diflunisal (Rissly, Ness.; Proc. Soc. Exp. Biol. Med., *111*, 544—547, 1962).

The activities inhibiting the carrageenin oedema in the rat paw are reported in Table 1.

MR 883 proved to be the most effective compound in the series, showing an antiinflammatory activity comparable to the one of Diflunisal.

$ED_{50}$ of MR 883 and Diflunisal were determined according to the same test. Groups of 10 rats were administered orally with the compounds at dosages of 25.50 and 100 mg/kg.

MR 883 proved to be substantially more effective, having an $ED_{50}$ of 145 mg/kg (ranging from 143.6 to 146.5 mg/kg), whilst the $ED_{50}$ of Diflunisal was 234 mg/kg (ranging from 224.9 to 243.5 mg/kg).

Analgesic activity

The analgesic activity of the compounds of the invention was determined according to the writhing test, as inhibitory activity against the writhing induced by phenylquinone administration in Swiss rats (Siegmund et al., Proc. Soc. Exp. Biol. Med. *95,* 729, 1957).

The analgesic activity of MR 883 was determined in comparison to the one of Diflunisal and Naproxen.

The results, reported in Table 2, clearly show that all the compounds of the invention have good analgesic activity, the most effective one being MR 883, which is substantially more active than Naproxen and Diflunisal.

TABLE N. 1

Antiinflammatory activity — Carrageenin induced oedema in rat

| TREATMENT | DOSE mg/kg p.o. | No. animals | AREAS | |
|---|---|---|---|---|
| | | | % M ± ES | Inhibition versus control |
| CONTROL | 10 ml | 10 | 305 ±23.7 | — |
| DIFLUNISAL | 50 | 10 | 123.3 ±10.1 | 59.6 |
| MR 864 | 50 | 10 | 174.7 ±20.7 | 42.7 |
| MR 881 | 50 | 10 | 156.8 ±13.1 | 48.6 |
| MR 883 | 50 | 10 | 144.0 ±17.5 | 52.8 |
| MR 936 | 50 | 10 | 139.7 ±15.2 | 45.8 |

TABLE N. 2
Analgesic activity — Phenylquinone writhing in rat

| TREATMENT | DOSE mg/kg | No. animals | Weight g M ± ES | No. WRITHINGS | | % Inhibition versus control |
|---|---|---|---|---|---|---|
| | | | | $\overline{M}$ ± ES | Significance versus control | |
| CONTROL | 10 ml | 10 | 26.9<br>±0.4 | 24.5<br>±2.2 | — | — |
| NAPROXEN | 100 | 10 | 20.7<br>±1.4 | 4.0<br>±1.1 | p<0.001 | 84 |
| DIFLUNISAL | 50 | 10 | 30.3<br>±1.2 | 4.3<br>±1.6 | p<0.001 | 82 |
| MR 864 | 50 | 10 | 26.9<br>±0.7 | 3.6<br>±1.2 | p<0.001 | 85 |
| MR 881 | 50 | 10<br>10 | 33.0 ± 0.6<br>28.2 ± 0.8 | 1.8 ± 0.5<br>0.7 ± 0.2 | p<0.001<br>p<0.001 | 95 |
| MR 883 | 50 | 10<br>10 | 31.8 ± 0.8<br>31.4 ± 0.4 | 0.7 ± 0.5<br>0.1 ± 0.0 | p<0.001<br>p<0.001 | 98 |
| MR 936 | 50 | 10 | 32.6<br>±0.5 | 5.7<br>±0.8 | p<0.001 | 76.7 |

$ED_{50}$ in the analgesic activity

The $ED_{50}$ in the analgesic activity of MR 883, in comparison to Diflunisal, D-propoxyphene, phenylbutazone and acetylsalicylic acid was determined according to the test of inhibition of writhing induced by acetic acid in the rat (Sofia et al., J. Pharmacol. Expt. Therap. *186,* 646, 1973).

The $ED_{50}$ of MR 883 was 25 mg/kg per os, substantially lower than the ones of the reference compounds.

The results are reported in Table 3.

TABLE 3
$ED_{50}$ determination in the rat

| COMPOUND | $ED_{50}$ |
|---|---|
| MR 883 | 25 mg/kg |
| Diflunisal | 45 mg/kg |
| D-propoxyphene | 40 mg/kg |
| Phenylbutazone | 180 mg/kg |
| Acetylsalicylic acid | 330 mg/kg |

The invention also provides pharmaceutical compositions containing, as active ingredient, prefixed and therapeutically effective amounts of at least one of the compounds of formula I and/or salts thereof, in admixture with a pharmaceutically acceptable carrier or diluent to be administered orally, rectally, or parenterally.

Examples of such formulations are tablets, capsules, pills, powders, granules, syrups, suppositories, vials, ampoules, possible sustained release dosage forms, obtained for instance by microincapsulation, together with suitable excipients commonly used in pharmaceutical technique.

The preferred dose of the compounds I will vary, depending upon the weight of the patient and the severity of the disease; generally it will range from 50 to 500 mg, 2—3 times a day.

**Claims for the Contracting States: BE CH DE FR GB IT LI LU NL SE**

1. Compounds of general formula I

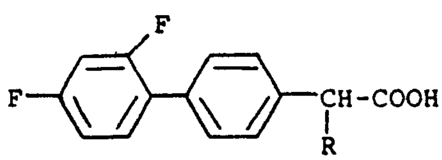

I

wherein R is hydroxy, methoxy, ethoxy or chlorine, pharmaceutically acceptable salts and enantiomers thereof.

2. Alkali, alkali-earth, lysine or arginine salts of the compounds according to claim 1.

3. 2-Hydroxy-2-[p-(2",4"-difluorophenyl)phenyl]acetic acid Ia.

4. 2-Chloro-2-[p-(2",4"-difluorophenyl)phenyl]acetic acid Ib.

5. 2-Methoxy-2-[p-(2",4"-difluorophenyl)phenyl]acetic acid Ic.

6. 2-Ethoxy-2-[p-(2",4"-difluorophenyl)phenyl]acetic acid Id.

7. Pharmaceutical compositions having analgesic and antiinflammatory activity characterized by containing as the active ingredient one or more compounds of claims 1—6.

8. Pharmaceutical compositions according to claim 7 in form of capsules, tablets, syrups, suppositories, creams, lotions, ointments, vials for injection.

9. Process for the preparation of compounds of formula I characterized in that:

a) 2,4-difluorobiphenyl is reacted with a monoester of oxalic acid chloride in the presence of Lewis acids;

b) the so obtained product is reduced to alcohol by means of suitable reducing agents such as hydrides or borohydrides of alkali metals and the ester group is hydrolized.

10. Process for the preparation of 2-chloro-2-[p-(2",4"-difluorophenyl)phenyl]acetic acid characterized

in that the 2-hydroxy-2-[p-(2",4"-difluorophenyl)phenyl]acetic acid is treated with thionylchloride in anhydrous toluene.

11. Process for the preparation of 2-methoxy-2-[p-(2",4"-difluorophenyl)phenyl]acetic acid or of 2-ethoxy-2-[p-(2",4"-difluorophenyl)phenyl]acetic acid characterized in that the 2-chloro-2-[p-(2",4"-difluorophenyl)phenyl]acetic acid is reacted with sodium methoxide or ethoxide in methanol or ethanol, respectively.

**Claims for the Contracting State: AT**

1. Process for the preparation of compounds of formula I

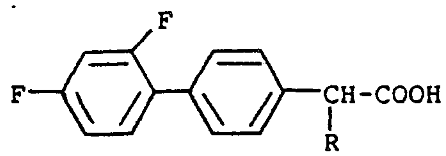

I

wherein R is hydroxy, methoxy, ethoxy or chlorine, pharmaceutically acceptable salts and enantiomers thereof, characterized in that:

a) 2,4-difluorobiphenyl is reacted with a monoester of oxalic acid chloride in the presence of Lewis acids;

b) the so obtained product is reduced to alcohol by means of suitable reducing agents such as hydrides or borohydrides of alkali metals and the ester group is hydrolized.

2. Process for the preparation of 2-chloro-2-[p-(2",4"-difluorophenyl)phenyl]acetic acid characterized in that the 2-hydroxy-2-[p-(2",4"-difluorophenyl)phenyl]acetic acid is treated with thionylchloride in anhydrous toluene.

3. Process for the preparation of 2-methoxy-2-[p-(2",4"-difluorophenyl)phenyl]acetic acid or of 2-ethoxy-2-[p-(2",4"-difluorophenyl)phenyl]acetic acid characterized in that the 2-chloro-2-[p-(2",4"-difluorophenyl)phenyl]acetic acid is reacted with sodium methoxide or ethoxide in methanol or ethanol, respectively.

**Patentansprüche für die Vertragsstaaten: BE CH DE FR GB IT LI LU NL SE**

1. Verfahren zur Herstellung von Verbindungen der allgemeinen Formel I

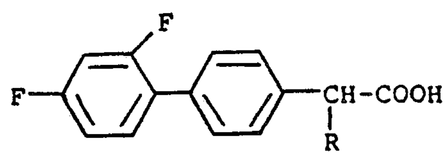

I

worin R Hydroxy, Methoxy, Ethoxy, oder Chlor ist, deren pharmazeutisch annehmbare Salze und Enantiomere.

2. Alkali-, Erdalkali-, Lysin- oder Argininsalze der Verbindungen gemäß Anspruch 1.

3. 2-Hydroxy-2-[p-(2",4"-difluorphenyl)phenyl]-essigsäure Ia.

4. 2-Chlor-2-[p-(2",4"-difluorphenyl)phenyl)-essigsäure Ib.

5. 2-Methoxy-2-[p-(2",4"-difluorphenyl)phenyl]-essigsäure Ic.

6. 2-Ethoxy-2-[p-(2",4"-difluorphenyl)phenyl]-essigsäure Id.

7. Pharmazeutisch Zusammensetzungen mit analgetischer und entzündungshemmender Aktivität, gekennzeichnet durch einen Gehalt einer oder mehrerer Verbindungen der Ansprüche 1 bis 6 als aktiver Bestandteil.

8. Pharmazeutische Zusammensetzungen nach Anspruch 7 in Form von Kapseln, Tabletten, Sirupen, Suppositorien, Cremes, Lotionen, Salben, Injektionsampullen.

9. Verfahren zur Herstellung von Verbindungen der Formel I, dadurch gekennzeichnet, daß

a) 2,4-Difluorbiphenyl mit einem Monoester von Oxalsäurechlorid in Gegenwart von Lewis-Säuren umgesetzt wird;

b) das so erhaltene Produkt durch geeignete Reduktionsmittel, wie Hydride oder Borhydride von Alkalimetallen zu Alkohol reduziert und die Estergruppe hydrolysiert wird.

10. Verfahren zur Herstellung von 2-Chlor-2-[p-(2",4"-difluorphenyl)phenyl]essigsäure, dadurch gekennzeichnet, daß die 2-Hydroxy-2-[p-(2",4"-difluorphenyl)phenyl]essigsäure mit Thionylchlorid in wasserfreiem Toluol behandelt wird.

11. Verfahren zur Herstellung von 2-Methoxy-2-[p-(2″,4″-difluorphenyl)phenyl]essigsäure oder von 2-Ethoxy-2-[p-(2″,4″-difluorphenyl)phenyl]essigsäure, dadurch gekennzeichnet, daß die 2-Chlor-2-[p-(2″,4″-difluorphenyl)phenyl]essigsäure mit Natriummethylat oder -ethylat in Methanol bzw. Ethanol umgesetzt wird.

**Patentansprüche für den Vertragsstaat: AT**

1. Verfahren zur Herstellung von Verbindungen der allgemeinen Formel I

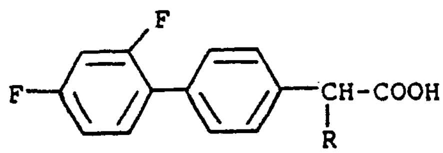

I

worin R Hydroxy, Methoxy, Ethoxy oder Chlor ist, deren pharmazeutisch annehmbare Salze und Enantiomere dadurch gekennzeichnet, dass

a) 2,4-Difluorbiphenyl mit einem Monoester von Oxalsäurechlorid in Gegenwart von Lewis-Säuren umgesetzt wird;

b) das so erhaltene Produkt durch geeignete Reduktionsmittel, wie Hydride oder Borhydride von Alkalimetallen zu Alkohol reduziert und die Estergruppe hydrolysiert wird.

2. Verfahren zur Herstellung von 2-Chlor-2-[p-(2″,4″-difluorphenyl)phenyl]essigsäure, dadurch gekennzeichnet, dass die 2-Hydroxy-2-[p-(2″,4″-difluorphenyl)hphenyl]essigsäure mit Thionylchlorid in wasserfreiem Toluol behandelt wird.

3. Verfahren zur Herstellung von 2-Methoxy-2-[p-(2″,4″-difluorphenyl)phenyl]essigsäure oder von 2-Ethoxy-2-[p-(2″,4″-difluorphenyl)phenyl]essigsäure, dadurch gekennzeichnet, dass die 2-Chlor-2-[p-(2″,4″-difluorphenyl)phenyl]essigsäure mit Natriummethylat oder -ethylat in Methanol bzw. Ethanol bzw. Ethanol umgesetzt wird.

**Revendications pour les Etats contractants: BE CH DE FR GB IT LI LU NL SE**

1. Composés de formule générale

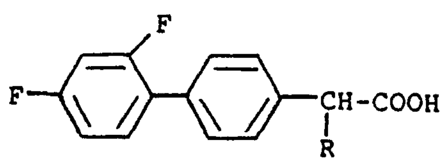

I

dans laquelle R est un groupe hydroxy, méthoxy ou éthoxy ou un atome de chlore, leurs sels pharmaceutiquement acceptables et leurs énantiomères.

2. Sels de métal alcalin, de métal alcalino-terreux, de lysine ou d'arginine des composés selon la revendication 1.

3. Acide 2-hydroxy-2-[p-(2″,4″-difluorophényl)phényl)-acétique (Ia).

4. Acide 2-chloro-2-[p-(2″,4″-difluorophényl)phényl]-acétique (Ib).

5. Acide 2-méthoxy-2-[p-(2″,4″-difluorophényl)phényl]-acétique (Ic).

6. Acide 2-éthoxy-2-[p-(2″,4″-difluorophényl)phényl]-acétique (Id).

7. Compositions pharmaceutiques à activité analgésique et antiinflammatoire, caractérisées en ce qu'elles contiennent à titre de principe(s) actif(s) un ou plusieurs des composés selon les revendications 1—6.

8. Compositions pharmaceutiques selon la revendication 7, sous la forme de capsules, de comprimés, de sirops, de suppositoires, de crèmes, de lotions, d'onguents ou de solutions injectables.

9. Procédé de préparation des composés de formule (I), caractérisé en ce que:

a) du 2,4-difluorobiphényle est mis à réagir avec un monoester du chlorure d'acide oxalique en présence d'acides de Lewis;

b) le composé ainsi obtenu est réduit en alcool au moyen d'agents réducteurs appropriés tels que des hydrures ou des borohydrures de métaux alcalins et le groupe ester est hydrolysé.

10. Procédé de préparation de l'acide 2-chloro-2-[p-(2″,4″-difluorophényl)phényl]acétique, caractérisé en ce que de l'acide 2-hydroxy-2-[p-(2″,4″-difluorophényl)phényl]acétique est traité par le chlorure de thionyle dans le toluène anhydre.

11. Procédé de préparation de l'acide 2-méthoxy-2-[p-2″,4″-difluorophényl)phényl]acétique ou de l'acide 2-éthoxy-2-[p-(2″,4″-difluorophényl)phényl]acétique, caractérisé en ce que l'acide 2-chloro-2-[p-(2″,4″-difluorophényl)phényl]acétique est améné en réaction avec du méthanolate ou de l'éthanolate de sodium dans le méthanol ou l'éthanol, respectivement.

**Revendications pour l'Etat contractant: AT**

1. Procédé de préparation des composés de formule (I):

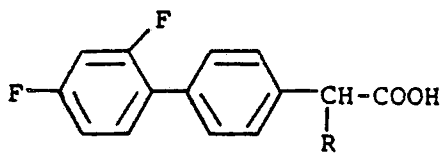

I

dans laquelle R est un groupe hydroxy, méthoxy ou éthoxy ou un atome de chlore, leurs sels pharmaceutiquement acceptables et leurs énantiomères, caractérisé en ce que:

a) du 2,4-difluorobiphényle est mis à réagir avec un monoester du chlorure d'acide oxalique en présence d'acides de Lewis;

b) le composé ainsi obtenu est réduit en alcool au moyen d'agents réducteurs appropriés tels que des hydrures ou des borohydrures de métaux alcalins et le groupe ester est hydrolysé.

2. Procédé de préparation de l'acide 2-chloro-2-[p-(2″,4″-difluorophényl)phényl]acétique, caractérisé en ce que de l'acide 2-hydroxy-2-[p-(2″,4″-difluorophényl)phényl]acétique est traité par le chlorure de thionyle dans le toluène anhydre.

3. Procédé de préparation de l'acide 2-méthoxy-2-[p-2″,4″-difluorophényl)phényl]acétique ou de l'acide 2-éthoxy-2-[p-(2″,4″-difluorophényl]phényl]acétique, caractérisé en ce que l'acide 2-chloro-2-[p-(2″,4″-difluorophényl)phényl]acétique est amené en réaction avec du méthanolate ou de l'éthanolate de sodium dans le méthanol ou l'éthanol, respectivement.